# EUROPEAN PATENT APPLICATION

(11) **EP 4 257 601 A1**
(43) Date of publication of application: **11.10.2023**
(21) Application number: 21903366.9
(22) Date of filing: 06.12.2021
(51) Int. Cl.: C07K 16/18, G01N 33/543, G01N 33/574, G01N 27/62

(54) **BREAST CANCER DIAGNOSIS ASSISTANCE METHOD AND BREAST CANCER TEST KIT**

(30) Priority: 07.12.2020 JP 2020202742
(71) Applicant: National Cancer Center, Tokyo 104-0045 (JP); Shimadzu Corporation, Nakagyo-ku, Kyoto-shi, Kyoto 604-8511 (JP); Dai Nippon Toryo Co., Ltd., Osaka-shi, Osaka, 542-0081 (JP)
(72) Inventor: TAMURA, Kenji, Tokyo 104-0045 (JP); SUDO, Kazuki, Tokyo 104-0045 (JP); MATSUDA, Takahisa, Tokyo 104-0045 (JP); YAMAJI, Taiki, Tokyo 104-0045 (JP); OKAMOTO, Koji, Tokyo 104-0045 (JP); WATANABE, Makoto, Kyoto-shi, Kyoto 604-8511 (JP); FUJIMOTO, Hirotaka, Kyoto-shi, Kyoto 604-8511 (JP); SATO, Taka-Aki, Kyoto-shi, Kyoto 604-8511 (JP); MIYAZAWA, Yuta, Otawara-shi, Tochigi 324-8516 (JP)
(74) Representative: Mewburn Ellis LLP
(86) International application number: PCT/JP2021/044729
(87) International publication number: WO 2022/124266

(57) **Abstract**

Provided is a method of assisting breast cancer diagnosis, comprising: a measuring step for measuring an amount of laminin 5 or laminin β3 in a specimen; and an information-providing step for providing information for breast cancer diagnosis based on the amount of laminin 5 or laminin β3 thus measured. Provided is a test kit for breast cancer comprising an anti-laminin 5 antibody or an anti-laminin β3 antibody.

## Description

### TECHNICAL FIELD

The present invention relates to a method of assisting breast cancer diagnosis and a test kit for breast cancer.

### BACKGROUND ART

As a breast cancer test method, visual inspection and palpation, mammography, ultrasonography, and the like are mainly employed. For those who are suspected of having cancer by these tests, a lesion sample is collected to conduct pathological examination by means of cytological testing and/or histological testing (biopsy) (NPL 1: Breast cancer, test and diagnosis, subtyping [searched on October 5, 2020] Internet <URL: https://ganjoho.jp/public/cancer/breast/diagnosis.html>, and NPL 2: Nover AB, Jagtap S, Anjum W, Yegingil H, Shih WY, Shih WH, Brooks AD. Int J Biomed Imaging. 2009, 902326).

For breast cancer subtyping, a classification method based on genetic testing is proposed (NPL 3: Garrido-Castro AC, Lin NU, Polyak K. Cancer Discov. 2019, 9(2): 176-198). However, practical use of genetic testing is considered to be difficult to achieve due to the cost. At this point, instead of performing genetic testing, cancer cells collected by biopsy and/or surgery are immunostained and judged clinicopathologically according to genetic analysis classification. In the immunostaining, hormone receptors (estrogen receptor (ER) and progesterone receptor (PgR)), HER2, and Ki67 are stained. These proteins are involved in cancer cell proliferation. Breast cancer subtyping is carried out when a diagnosis of breast cancer is established. Triple-negative-type breast cancer progresses rapidly with poor prognosis as compared to other subtypes, so it would be useful if triple-negative-type breast cancer could be detected in an early stage by blood testing.

### CITATION LIST

### NON PATENT LITERATURE

NPL 1: Breast cancer, test and diagnosis, subtyping [searched on October 5, 2020] Internet <URL: https://ganjoho.jp/public/cancer/breast/diagnosis.html>
NPL 2: Nover AB, Jagtap S, Anjum W, Yegingil H, Shih WY, Shih WH, Brooks AD. Int J Biomed Imaging. 2009, 902326
NPL 3: Garrido-Castro AC, Lin NU, Polyak K. Cancer Discov. 2019, 9(2):176-198

### SUMMARY OF INVENTION

### TECHNICAL PROBLEM

For early detection and proper treatment of breast cancer, easy and simple testing for breast cancer is desirable. An object of the present invention is to provide a new method of assisting breast cancer diagnosis and a new test kit for breast cancer.

### SOLUTION TO PROBLEM

The present invention relates to a method of assisting breast cancer diagnosis, comprising: a measuring step for measuring an amount of laminin 5 or laminin β3 contained in a specimen; and an information-providing step for providing information for breast cancer diagnosis based on the amount of laminin 5 or laminin β3 thus measured.

The present invention relates to a test kit for breast cancer comprising an anti-laminin 5 antibody or an anti-laminin β3 antibody.

### ADVANTAGEOUS EFFECTS OF INVENTION

According to the present invention, a new method of assisting breast cancer diagnosis and a new test kit for breast cancer are provided.

### BRIEF DESCRIPTION OF DRAWINGS

Fig. 1 is a descriptive view of the structure of laminin.
Fig. 2 is a view illustrating an example of a test kit for breast cancer according to the present invention.
Fig. 3 is a view illustrating an example of use of a test kit for breast cancer according to the present invention.
Fig. 4 is a graph showing the amount of laminin 5 expression in a cell line derived from breast cancer in Experiment 2 in Examples.
Fig. 5 shows, on the left, results of detection, by an immunochromatography method, of the amounts of laminin 5 and CD9 (an exosome marker) in plasma from breast cancer patients and healthy individuals in Experiment 3 in Examples; and, on the right, a graph showing the amount of laminin 5 expression standardized based on the amount of CD9 expression (laminin 5/CD9), from the immunochromatography results provided on the left.
Fig. 6 is a graph showing the amount of laminin 5 in plasma from HER2-type breast cancer patients and triple-negative-type breast cancer patients standardized based on the amount of CD9 (laminin 5/CD9) in Experiment 4 in Examples.
Fig. 7A is a view showing the amount of laminin 5 in breast cancer patients and healthy individuals standardized based on the amount of CD9 (laminin 5/CD9) in Experiment 5 in Examples.
Fig. 7B shows an ROC curve indicating the capability of laminin 5 of identifying breast cancer patients in Experiment 5 in Examples.
Fig. 8A is a view showing the amount of laminin 5 in early-stage breast cancer patients and healthy individuals standardized based on the amount of CD9 (laminin 5/CD9) in Experiment 5 in Examples.
Fig. 8B shows an ROC curve indicating the capability of laminin 5 of identifying early-stage breast cancer patients in Experiment 5 in Examples.
Fig. 9A is a view showing the amount of laminin 5 in breast cancer patients and healthy individuals standardized based on the amount of CD9 (laminin 5/CD9) in Experiment 6 in Examples.
Fig. 9B shows an ROC curve indicating the capability of laminin 5 of identifying breast cancer patients in Experiment 6 in Examples.

### DESCRIPTION OF EMBODIMENTS

### <Method of Assisting Breast Cancer Diagnosis>

A method of assisting breast cancer diagnosis according to an aspect of the present invention comprises:
a measuring step for measuring an amount of laminin 5 or laminin β3 contained in a specimen; and
an information-providing step for providing information for breast cancer diagnosis based on the amount of laminin 5 or laminin β3 thus measured.

With the method of assisting breast cancer diagnosis according to the present invention, it is possible to provide information for breast cancer diagnosis in an easy and simple manner. The diagnosis assistance method according to the present invention does not comprise a step of judgement performed by a doctor, so it does not fall under the category of a method of diagnosis of a human.

In the following, each step will be described in detail.

The method of assisting breast cancer diagnosis according to the present invention firstly measures the amount of laminin 5 or laminin β3 contained in a specimen. Hereinafter, this step is also called "a measuring step".

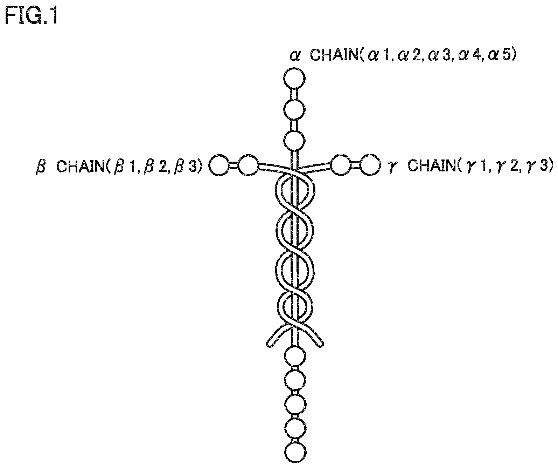

Laminin is a large-sized protein serving as a component of the basal lamina which is an extracellular matrix, and it is involved with building and maintaining multicellular configuration and tissue, and with cell adhesion, cell migration, cell proliferation facilitation, and the like. With reference to Fig. 1, laminin has a heterotrimer structure composed of three polypeptide chains, namely α chain, β chain, and γ chain, bonded via disulfide bonding. In humans, there are five types of α chains (α1, α2, α3, α4, α5) as well as three types of β chains (β1, β2, β3) and γ chains (γ1, γ2, γ3), and, to date, at least 17 types of laminin have been identified which have different combinations of these. Laminin 5, which is also called laminin-332, is composed of α3 chain, β3 chain, and γ2 chain. Laminin β3 is only contained in laminin 5, among all laminins reported so far. The amount of laminin 5 can correlate with the amount of laminin β3. It is believed that laminin β3 contained in a specimen includes both laminin β3 in the form of a trimer complex as well as laminin β3 not in the form of a complex.

The method for measuring the amounts of laminin 5 and laminin β3 is not particularly limited, and a known method capable of accurate quantification of proteins can be used. Examples of the measurement method include an immunological measurement method, a mass spectrometry method, and the like. These measurement methods may be combined as appropriate.

An immunological measurement method is a method of measurement using antigen-antibody reaction, and examples include immunochromatography method, enzyme immunoassay method (Enzyme Immunoassay (EIA), Enzyme-Linked Immuno Sorbent Assay (ELISA)), chemiluminescence enzyme immunoassay method, fluorescence immunoassay method, radioimmunoassay method, immunoturbidimetry method, nephelometric immunoassay method, latex agglutination method, immunoprecipitation method, Western blotting, affinity chromatography method, flow cytometry, an immunoassay method using a combination of metal-labelled antibody and mass spectrometry, and the like. From the viewpoint of fast, easy, and simple measurement, immunochromatography method is preferred.

The antibody used in the immunological measurement method includes an anti-laminin 5 antibody or an anti-laminin β3 antibody. An anti-laminin 5 antibody and an anti-laminin β3 antibody are used in detection of laminin 5 and laminin β3, respectively. From the viewpoint of excellent detection sensitivity and specificity, an anti-laminin 5 antibody is preferably used in the immunological measurement method.

The antibody used in the immunological measurement method may be labelled with a substance suitable for detection. Examples of the label include metal particles, enzymes, fluorescent substances, radioactive substances, affinity substances, and the like. Examples of the metal particles include gold nanoparticles, silver nanoparticles, gold nanorods, silver nanorods, gold nanoplates, silver nanoplates, as well as metal nanoparticles of various shapes coated with a different type of metal. Examples of the enzymes include peroxidase, alkaline phosphatase, luciferase, β galactosidase, and the like. Examples of the fluorescent substances include Fluorescein Isothiocyanate (FITC), Cy dye, Alexa Fluor dye, rhodamine, Phycoerythrin (PE), Allophycocyanin (APC), other fluorescent proteins, and the like. Examples of the radioactive substances include ³H, ¹⁴C, ³²P, ³⁵S, ¹²⁵I, and the like. Examples of the affinity substances include biotin, streptavidin, and the like. The label may be attached to the anti-laminin 5 antibody or the anti-laminin β3 antibody, or may be attached to a secondary antibody that is capable of specifically recognizing these primary antibodies. From the viewpoint of highly-sensitive detection, the antibody is preferably labelled with metal particles or with an enzyme. Chemiluminescence emitted by a substrate through catalytic effect of the enzyme amplifies the signal, leading to an enhanced detection sensitivity. The absorption wavelength of an antibody labelled with metal particles changes upon aggregation, allowing for checking the amount of laminin 5 or laminin β3 as a change of color detectable by the naked eye. The change of color may be converted into numerical values with the use of a known image processing apparatus. An antibody labelled with metal particles is also detectable by inductively coupled plasma mass spectrometry (ICP-MS), allowing for highly-sensitive measurement even when the amount of laminin 5 or laminin β3 is low. Antibody detection may be carried out with the use of an apparatus usually used for detection of the label.

As an example of a method for measuring laminin 5 or laminin β3 by an immunological measurement method, a specimen may be subjected to immunochromatography for development (migration) and capture, followed by detection of the labelled anti-laminin 5 antibody or anti-laminin β3 antibody by means of visible light (absorption), ICP-MS, chemiluminescence, or fluorescence.

Examples of the method for measuring the amount of laminin 5 and laminin β3 by a mass spectrometry method include ionizing a protein by an ionization method such as matrix-assisted laser desorption ionization (MALDI) method and/or electrospray ionization (ESI) method, and then using the signal intensities of peaks separated according to the mass/charge ratio (m/z) of ions of the protein, to perform quantitative analysis. Alternatively, quantitative analysis of the target protein may be carried out by peptide quantification by means of a liquid chromatography-mass spectrometry method (LC-MS), which involves enzymatically digesting the specimen with a protease such as trypsin and then separating and concentrating the resulting peptide fragments by liquid chromatography (LC), followed by mass spectrometry. As the mass spectrometer, a typically-used single mass spectrometer, as well as a tandem mass spectrometer such as a triple quadrupole (QqQ) mass spectrometer, a quadrupole time-of-flight (Q-TOF) mass spectrometer, a tandem time-of-flight (TOF-TOF) mass spectrometer, a quadrupole ion trap (QIT) mass spectrometer, and/or a quadrupole ion trap/time-of-flight (QIT/TOF) mass spectrometer can be preferably used. In LC-MS/MS, a triple quadrupole mass spectrometer may be used to perform multiple reaction monitoring (MRM). A triple quadrupole mass spectrometer is excellent in quantification capability. In MRM, in which separation from contaminant ions takes place inside the apparatus, the noise level can be further lowered, allowing for quantitative analysis of trace peptides.

In the case when the amount of laminin 5 or laminin β3 is measured by a mass spectrometry method, in which dissociation of protein complexes and fragmentation of proteins may occur, it is preferable to measure the amount of laminin β3.

The specimen may be cultured cells, cultured tissue, or the resulting culture supernatant, and, preferably, it is a biological specimen collected from a subject. The biological specimen may be blood collected from an animal, and may be biological tissue, urine, stool, sweat, saliva, lymph fluid, amniotic fluid, breast milk, pleural fluid, ascitic fluid, cerebrospinal fluid, lacrimal fluid, and/or the like. Blood may be whole blood, and, preferably, it is plasma or serum. The specimen may be diluted with physiological saline, buffer, and/or the like. The biological specimen may be collected by a known method which is appropriate for the type of the biological specimen. The subject is preferably a human individual, such as, for example, a breast cancer patient, a suspected breast cancer patient, and/or a healthy individual. By choosing a biological specimen collected from a subject as the specimen, it is possible to provide information for breast cancer diagnosis of the subject. The specimen is preferably blood collected from a human individual. By using blood collected from a human individual as the specimen, it is possible to provide information for breast cancer diagnosis of the human in an accurate, easy, and simple manner.

For more accurate measuring of the amount of laminin 5 and laminin β3, the specimen may be purified or pretreated as appropriate, and may be subjected to filtration, centrifugation, affinity separation, dialysis, sedimentation treatment, and/or the like. The specimen may be a purified exosome fraction. When the specimen is a purified exosome fraction, the amount of foreign matter in the specimen may be lowered, leading to enhanced accuracy of the measurement. Exosomes are vesicles of a diameter of around 100 nm secreted by cells, and present in abundance in body fluid such as blood, saliva, and urine. It is known that exosomes have on their surfaces lipids and proteins derived from cell membrane and also contain nucleic acids such as mRNA and miRNA as well as proteins. Purification of exosomes can be carried out by a known method such as use of a commercially available purification kit, centrifugation, density gradient centrifugation, size exclusion chromatography, filtering treatment, and/or the like.

The method of assisting breast cancer diagnosis according to the present invention includes providing information for breast cancer diagnosis based on the amount of laminin 5 or laminin β3 thus measured. Hereinafter, this step is also called "an information-providing step".

In the information-providing step, the amount of laminin 5 or laminin β3 contained in the specimen is compared to a predetermined value, to obtain information for breast cancer diagnosis. The information for breast cancer diagnosis may include information for diagnosis of, for example, (1) presence or absence of breast cancer, (2) breast cancer subtype, (3) breast cancer stage, and/or the like. One, two, or more types of these information may be provided at the same time.

### (1) Presence or Absence of Breast Cancer

The 5-year survival rate of a breast cancer patient decreases as the stage progresses, so it is preferable to establish diagnosis as early as possible. With the diagnosis assistance method according to the present embodiment, it is possible to provide information for diagnosis of presence or absence of breast cancer, even it is early-stage breast cancer which is difficult to find by conventional testing, in a non-invasive, easy, and simple manner.

The amount of laminin 5 or laminin β3 relative to a predetermined value may be provided as information that is useful for diagnosis of presence or absence of breast cancer. The predetermined value is, for example, the amount of laminin 5 or laminin β3 in culture supernatant from cultured cells that are not derived from breast cancer, and/or the amount of laminin 5 or laminin β3 in body fluid (such as plasma, serum, and/or the like) from a healthy individual. In a breast cancer patient, as compared to a healthy individual, more laminin 5 or laminin β3 tends to be detected. When the amount of laminin 5 or laminin β3 contained in the specimen is higher than the predetermined value, this information is useful for judging that the specimen is derived from breast cancer.

### (2) Breast Cancer Subtype

As breast cancer subtypes, luminal-A type, luminal-B type, HER2 type, and triple-negative-type are known. Triple-negative-type breast cancer is negative for all of estrogen receptor (ER), progesterone receptor (PgR), and Human Epidermal Growth Factor Receptor 2 (HER2). For treating breast cancer, a method has been employed which involves classifying the breast cancer into a specific subtype and selecting a therapy suitable for the characteristics of the cancer cells, such as, for example, endocrine (hormone) therapy, chemotherapy, molecular targeted therapy, and/or the like. Hence, for performing proper treatment for breast cancer, diagnosis of breast cancer subtype is necessary.

Breast cancer subtyping is performed at the time when breast cancer is suspected by testing, so it tends not to be performed for a relatively early-stage cancer patient. For breast cancer subtyping, classification by genetic testing is suggested. However, practical use of genetic testing is difficult to achieve due to the cost. In the case of triple-negative-type breast cancer, it progresses rapidly, with a low 5-year relapse-free survival rate for late-stage (Stages III and IV) triple-negative-type breast cancer patients. For this reason, it is necessary to establish diagnosis of breast cancer subtype as early as possible. With the diagnosis assistance method according to the present embodiment, it is possible to provide information for diagnosis of breast cancer subtype in an easy and simple manner.

The amount of laminin 5 or laminin β3 relative to a predetermined value may be provided as information that is useful for diagnosing breast cancer subtype, particularly triple-negative-type breast cancer. The predetermined value is, for example, the amount of laminin 5 or laminin β3 in culture supernatant from cultured cells that are not derived from breast cancer or from cultured cells that are not derived from triple-negative-type breast cancer, and/or the amount of laminin 5 or laminin β3 in body fluid (such as plasma, serum, and/or the like) from a healthy individual or a patient of breast cancer other than triple-negative-type breast cancer. In a triple-negative-type breast cancer patient, as compared to a healthy individual and a patient of breast cancer other than triple-negative-type breast cancer, more laminin 5 or laminin β3 tends to be detected. When the amount of laminin 5 or laminin β3 contained in the specimen is higher than the predetermined value, this information is useful for judging that the specimen is derived from triple-negative-type breast cancer.

### (3) Breast Cancer Stage

The extent of progress of breast cancer is classified into Stages 0, I, II (IIA, IIB), III (IIIA, IIIB, IIIC), and IV, depending on the size of lump and/or the extent of metastasis to lymph nodes or other organs. Because the treatment is performed depending on the extent of progress of the cancer, it is necessary to diagnose breast cancer stage. With the diagnosis assistance method according to the present embodiment, it is possible to provide information for diagnosis of breast cancer stage in an easy and simple manner.

The amount of laminin 5 or laminin β3 relative to a predetermined value may be provided as information that is useful for diagnosing breast cancer stage, particularly early-stage breast cancer at Stages I and II. For this purpose, the predetermined value is, for example, the amount of laminin 5 or laminin β3 in culture supernatant from cultured cells that are not derived from breast cancer or from cultured cells derived from late-stage breast cancer at Stage III or Stage IV, and/or the amount of laminin 5 or laminin β3 in body fluid (such as plasma, serum, and/or the like) from a healthy individual or a late-stage breast cancer patient at Stage III or Stage IV. In an early-stage breast cancer patient, as compared to a healthy individual or a late-stage breast cancer patient, more laminin 5 or laminin β3 tends to be detected. When the amount of laminin 5 or laminin β3 contained in the specimen is higher than the predetermined value, this information is useful for judging that the specimen is derived from early-stage breast cancer.

In the information-providing step, the amount of laminin 5 or laminin β3 may be an actual measured value or may be the concentration in the specimen, preferably the amount of laminin 5 or laminin β3 relative to the amount of a control contained in the specimen. The control may be exosomes, for example. When exosomes are used as a control, the amount of laminin 5 or laminin β3 is standardized based on the amount of exosomes in the information-providing step. Because the amount of exosomes contained in a sample may vary between multiple samples, standardization of the measured amount of laminin 5 or laminin β3 based on the control can enhance the accuracy of the information for breast cancer diagnosis. The amount of exosomes can be measured by means of the amount of an exosome marker. Examples of the exosome marker include surface antigens such as CD9, CD63, CD81, and/or the like.

When the ratio of the amount of laminin 5 or laminin β3 to the amount of CD9 contained in the specimen is more than, preferably at least twice, the ratio of the amount of laminin 5 or laminin β3 to the amount of CD9 in plasma from a healthy individual, for example, this information is useful for judging that the specimen is derived from breast cancer including triple-negative-type breast cancer and/or that it is derived from early-stage breast cancer.

The amount of an exosome marker may be measured by a known method, preferably by the same method as the above-described method for measuring the amount of laminin 5 or laminin β3. In the case when the amount of an exosome marker is measured by an immunological measurement method, the amount of the exosome marker may be measured by the same method as the method for laminin 5 or laminin β3, by using an antibody against the exosome marker. When the antibody against the exosome marker is labelled with a substance that is different from those for the anti-laminin 5 antibody or the anti-laminin β3 antibody (different metal particles, different fluorescent substance, and/or the like), it is possible to detect both laminin 5 or laminin β3 and the exosome marker in the same experiment system. When a mass spectrometry method is employed, which can detect any protein, it is possible to measure the amount of the exosome marker and measure the amount of laminin 5 or laminin β3 in a single analysis.

### <Test Kit for Breast Cancer>

A test kit for breast cancer according to an aspect of the present invention comprises an anti-laminin 5 antibody or an anti-laminin β3 antibody. With the test kit for breast cancer according to the present invention, it is possible to provide information for breast cancer diagnosis in an easy and simple manner by measuring the amount of laminin 5 or laminin β3 contained in a specimen. The information for breast cancer diagnosis may include information for diagnosis of, for example, (1) presence or absence of breast cancer, (2) breast cancer subtype, (3) breast cancer stage, and/or the like. One, two, or more types of these information may be provided at the same time.

The anti-laminin 5 antibody or the anti-laminin β3 antibody may be the same antibody as used in the above-described method for measuring the amount of laminin 5 or laminin β3. The anti-laminin 5 antibody or the anti-laminin β3 antibody is used for detection of laminin 5 or laminin β3, and preferably labelled with metal particles or with an enzyme. The label may be attached to a secondary antibody that is to be used for detection of the anti-laminin 5 antibody or the anti-laminin β3 antibody. When the antibody is labelled with an enzyme, a signal attributable to a reaction product from the substrate is detected; so, by increasing the reaction time and/or the detection time, detection can become highly sensitive. When the antibody is labelled with metal particles, the absorption wavelength changes upon aggregation, making it possible to check the amount of laminin 5 or laminin β3 by means of visible light (absorption). Even when it is in a small amount, an antibody labelled with metal particles is detectable by inductively coupled plasma mass spectrometry (ICP-MS), allowing for highly-sensitive measurement of the amount of laminin 5 or laminin β3.

The test kit is preferably capable of measuring the amount of laminin 5 or laminin β3 contained in a specimen by an immunological measurement method or a mass spectrometry method. From the viewpoint of fast, easy, and simple measurement, the test kit preferably employs an immunochromatography method.

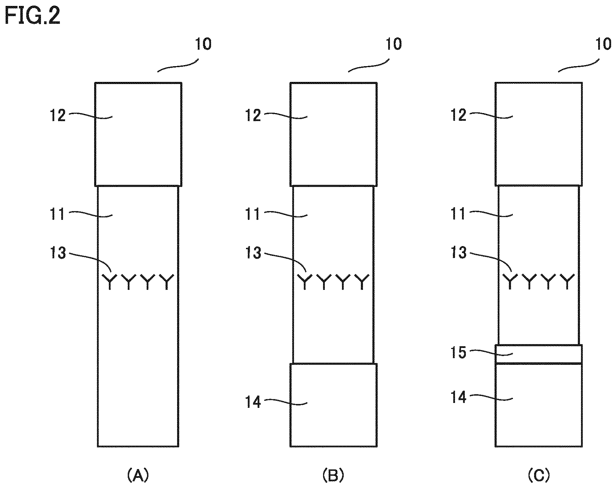

The test kit may further comprise an immunochromatographic test strip for performing immunochromatography, an antibody for detecting a control, a laminin 5 or laminin β3 capturing factor or antibody, a laminin 5 or laminin β3 reference material, an instruction manual, and/or the like. An example of the immunochromatographic test strip contained in the test kit according to the present invention is shown in Fig. 2. An immunochromatographic test strip 10 typically comprises a membrane 11 and a water-absorbing pad 12 connected to each other (Fig. 2A). On membrane 11, a capture antibody 13 may be fixed, forming a detection line. Capture antibody 13 may be a laminin 5 or laminin β3 capturing factor or antibody, or an exosome-marker-capturing factor or antibody. Membrane 11 is a nitrocellulose membrane, for example. Water-absorbing pad 12 is a part that is capable of quickly absorbing the specimen. Immunochromatographic test strip 10 may further have a sample pad 14 (Fig. 2B), or may have sample pad 14 and a conjugate pad 15 (Fig. 2C). Sample pad 14 is a part serving as a sample feed part, capable of absorbing a liquid specimen and allowing passage of liquid and a detection target. Conjugate pad 15 contains a detection antibody and is capable of releasing the detection antibody into membrane 11. The detection antibody may be a laminin 5 or laminin β3 capturing factor or antibody, or an exosome-marker-capturing factor or antibody. As a control, exosomes can be used. Exosomes can be detected by one of the marker proteins, CD9. The laminin 5 or laminin β3 capturing factor or antibody may be an anti-laminin 5 antibody or an anti-laminin β3 antibody fixed on the membrane. When laminin 5 or laminin β3 contained in exosomes is to be detected, the laminin 5 or laminin β3 capturing factor or antibody may be an exosome-marker-capturing antibody fixed on the membrane, and may be, for example, an anti-CD9 antibody.

For immunochromatography testing, capture antibody 13 and the detection antibody can be used in the combination specified in Table 1, for example. With reference to Pattern 1, by using an exosome-marker-capturing antibody as a capture antibody, and an exosome-marker-capturing antibody as a detection antibody, it is possible to detect exosomes present in the specimen, allowing for quantification of exosomes as a control. With reference to Pattern 2, by using an anti-laminin 5 antibody or an anti-laminin β3 antibody as a capture antibody, and an anti-laminin β3 antibody or an anti-laminin β3 antibody as a detection antibody, it is possible to detect laminin 5 or laminin β3 present in the specimen, allowing for quantification of laminin 5 or laminin β3. With reference to Pattern 3, by using an exosome-marker-capturing antibody as a capture antibody, and an anti-laminin 5 antibody or an anti-laminin β3 antibody as a detection antibody, it is possible to detect exosomes having laminin 5 or laminin β3 present in the specimen, allowing for quantification of laminin 5 or laminin β3 expressed in the exosomes. With reference to Pattern 4, by using an anti-laminin 5 antibody or an anti-laminin β3 antibody as a capture antibody, and an exosome-marker-capturing antibody as a detection antibody, it is possible to detect exosomes having laminin 5 or laminin β3 present in the specimen, allowing for quantification of laminin 5 or laminin β3 expressed in the exosomes.

**[Table 1]**

| Pattern | Antibody combination | | Purpose |
|---|---|---|---|
| | Capture antibody (fixed on membrane) | Detection antibody (labelled with detectable substance) | |
| 1 | Exosome-marker-capturing antibody (anti-CD9 antibody, etc.) | Exosome-marker-capturing antibody (anti-CD9 antibody, etc.) | Quantification of exosomes |
| 2 | Anti-laminin 5 antibody or anti-laminin β3 antibody | Anti-laminin 5 antibody or anti-laminin β3 antibody | Quantification of laminin 5 or laminin β3 |
| 3 | Exosome-marker-capturing antibody (anti-CD9 antibody, etc.) | Anti-laminin 5 antibody or anti-laminin β3 antibody | Quantification of laminin 5 or laminin β3 expressed in exosomes |
| 4 | Anti-laminin 5 antibody or anti-laminin β3 antibody | Exosome-marker-capturing antibody (anti-CD9 antibody, etc.) | Quantification of laminin 5 or laminin β3 expressed in exosomes |

The specimen to be tested may be the same specimen as used in the above-described method for measuring the amount of laminin 5 or laminin β3. The specimen is preferably blood, plasma, serum, or a purified exosome fraction from them. The specimen is preferably blood collected from a human individual.

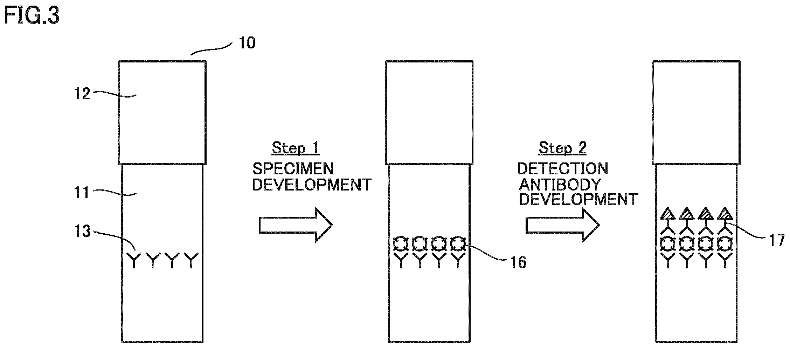

An example of use of the test kit according to the present invention is illustrated in Fig. 3. The test kit illustrated in Fig. 3 comprises immunochromatographic test strip 10. On the detection line on membrane 11 of immunochromatographic test strip 10, capture antibody 13 is fixed. The specimen is absorbed at an end of immunochromatographic test strip 10 opposite to water-absorbing pad 12, developed through membrane 11 by capillary action, and then absorbed into water-absorbing pad 12 (Step1). At this time, a detection target 16 (laminin 5 or laminin β3 or exosomes containing them) contained in the specimen is captured. After the development of the specimen, or simultaneously with the development of the specimen, a detection antibody 17 labelled with metal particles is developed (Step2). With detection antibody 17 thus labelled with metal particles, as detection antibody 17 moves to and comes together at the detection line, coloring at the detection line can be visible by visual examination. Detection antibody 17 labelled with metal particles can be detected by ICP-MS. When detection antibody 17 is labelled with an enzyme or fluorescence, the substrate can be added for detecting detection antibody 17 as chemiluminescence or fluorescence.

For better quantitative evaluation of laminin 5 or laminin β3, the amount of CD9 may be measured as a control and the amount of CD9 contained in the specimen may be used for standardizing the amount of laminin 5 or laminin β3. The amount of CD9 can be measured by an immunochromatography method in which an anti-CD9 antibody is used as a capture antibody and a detection antibody. Measurement of the amount of CD9 may be carried out by using the same kit as the test kit according to the present invention, or by using a different kit. Detection of CD9 and detection of laminin 5 or laminin β3 may be carried out at the same time, by capturing exosomes with the use of a capture antibody present on a single membrane and, then, subjecting the exosomes to detection with the use of a combination of an anti-CD9 antibody and an anti-laminin 5 antibody or an anti-laminin β3 antibody which are labelled with different substances.

The amount of laminin 5 or laminin β3 measured with the use of the test kit is compared with a predetermined value. The predetermined value may be a value mentioned in Section <Method of Assisting Breast Cancer Diagnosis>, and, for example, it is the amount of laminin 5 or laminin β3 in culture supernatant from cultured cells that are not derived from breast cancer, or from cultured cells that are not derived from triple-negative-type breast cancer, or from cultured cells derived from late-stage breast cancer, and/or the amount of laminin 5 or laminin β3 in body fluid (such as plasma, serum, and/or the like) from a healthy individual, or a patient of breast cancer other than triple-negative-type breast cancer, or a late-stage breast cancer patient. When it is detected, by the test kit, that the amount of laminin 5 or laminin β3 contained in the specimen is higher than the predetermined value, this information is useful for judging that the specimen is derived from breast cancer including triple-negative-type breast cancer and/or that it is derived from early-stage breast cancer.

When the ratio of the amount of laminin 5 or laminin β3 to the amount of a control measured by the test kit is higher than, preferably at least twice, the ratio of the amount of laminin 5 or laminin β3 to the amount of a control in plasma from a healthy individual (optional), this information is useful for judging that the specimen is derived from breast cancer including triple-negative type and/or that it is derived from early-stage breast cancer.

### <Breast Cancer Diagnostic Marker>

A breast cancer diagnostic marker according to an aspect of the present invention consists of laminin 5 or laminin β3 contained in a specimen. With the breast cancer diagnostic marker according to the present invention, it is possible to provide information for breast cancer diagnosis, and it is possible to provide information that is useful for diagnosis of presence or absence of breast cancer, breast cancer subtype, and breast cancer stage, in an easy and simple manner.

The specimen and laminin 5 or laminin β3 may be those as described above. The method for detecting the diagnostic marker according to the present invention is not particularly limited, and, for example, the above-described method may be employed for measuring the amount of laminin 5 or laminin β3. Based on the amount of laminin 5 or laminin β3 thus measured, and according to the above-described criteria, it is possible to judge if the specimen was derived from breast cancer, if it was derived from triple-negative-type breast cancer, and if it was derived from early-stage breast cancer.

### <Measurement Method>

A measurement method according to an aspect of the present invention involves measuring the amount of laminin 5 or laminin β3 contained in a specimen. With the measurement method according to the present invention, it is possible to determine the amount of laminin 5 or laminin β3 contained in a specimen. The amount of laminin 5 or laminin β3 can provide information for breast cancer diagnosis, and can be used as information for diagnosis of presence or absence of breast cancer, breast cancer subtype, and breast cancer stage. When the amount of laminin 5 or laminin β3 contained in the specimen is equal to or higher than a predetermined value, this information is useful for judging that the specimen is derived from breast cancer including triple-negative-type breast cancer and/or that it is derived from early-stage breast cancer.

The measurement method is not particularly limited provided that it is capable of measuring the amount of laminin 5 or laminin β3 contained in a specimen, and it may be the same method as mentioned above in the section regarding the measuring step. The specimen and the predetermined value may be those described above.

### <Breast Cancer Diagnostic Method>

A breast cancer diagnostic method according to an aspect of the present invention comprises judging that a specimen was derived from breast cancer when the amount of laminin 5 or laminin β3 contained in the specimen is equal to or higher than a predetermined value. It is also capable of diagnosing breast cancer subtype and breast cancer stage for the specimen. With the breast cancer diagnostic method according to the present invention, it is possible to diagnose if the specimen was derived from breast cancer, if it was derived from triple-negative-type breast cancer, and if it was derived from early-stage breast cancer, in an easy and simple manner.

The specimen and the predetermined value may be those described above. When the specimen is a biological specimen collected from a subject, it is possible, by the breast cancer diagnostic method according to the present invention, to judge if the subject is a breast cancer patient as well as to judge breast cancer subtype and breast cancer stage, in an easy and simple manner. The breast cancer diagnostic method according to the present invention may comprise the measuring step and the information-providing step as described above.

### [Examples]

In the following, the present invention will be described in more detail with reference to Examples, but it should be noted that the present invention is not limited to these Examples.

### <Experiment 1>

In order to investigate the difference between protein expression in triple-negative-type breast cancer and that in other breast cancers, exhaustive protein analysis was carried out. Firstly, from culture supernatant of a human triple-negative-type breast cancer cell line (MM231) and from culture supernatant of a luminal-A-type breast cancer cell line (MCF7), an exosome fraction was purified by ultracentrifugation. The resulting exosome fraction thus purified was enzymatically digested, and then subjected to shotgun proteomic analysis with the use of mass spectrometry (Orbitrap mass spectrometer) to detect proteins contained in the exosome fraction. Laminin β3 was detected in a high concentration in the triple-negative-type breast cancer cell line, while it was detected in a low concentration in the luminal-A-type breast cancer cell line. Since laminin β3 is contained only in laminin 5, it is suggested that laminin 5 is expressed in a high amount in the triple-negative-type breast cancer cell line and also contained in exosomes.

### <Experiment 2>

With the use of other human triple-negative-type breast cancer cell lines, expression of laminin 5 was investigated. From each of culture supernatants of 11 types of human triple-negative-type breast cancer cell lines as well as a luminal-A-type breast cancer cell line (MCF7), an exosome fraction was purified by ultracentrifugation, and the amount of laminin 5 expression and the amount of CD9 expression were detected by an immunochromatography method.

### (Detection of CD9 Expression Amount)

To the central part of a half strip of an immunochromatographic test strip having a nitrocellulose membrane, a mouse anti-CD9 antibody (manufactured by Hansa Bio Med LifeSciences, Product No. HBM-CD9-100) was fixed. Subsequently, the membrane was subjected to blocking with a water-soluble polymer and BSA. An end of the immunochromatographic test strip to which a water-absorbing pad was not attached was immersed in a solution containing 1×10⁹ exosomes, allowing them to develop through the immunochromatographic test strip. The exosome solution was prepared by diluting the exosome fraction obtained above by purification in Experiment 1, with PBS containing 1% BSA and 0.05% Tween 20. It was followed by developing a developer liquid containing mouse anti-CD9 antibody to which HRP had been conjugated with the use of Ab-10 Rapid Peroxidase Labeling Kit (manufactured by DOJINDO LABORATORIES, Product No. LK33). Then, luminescence was generated with the use of ImmunoStar (registered trademark) LD (manufactured by FUJIFILM Wako Pure Chemical Corporation, Product No. 290-69904), and the resulting luminescence was detected on Image Quant LAS4000.

### (Detection of Laminin 5 Expression Amount)

By the same procedure except that the above antibody was replaced by rabbit anti-LAMS antibody (manufactured by Abcam, Product No. ab 14509), a solution containing 1×10¹⁰ exosomes was developed. It was followed by developing a developer liquid containing rabbit anti-LAMS antibody to which HRP had been conjugated with the use of Ab-10 Rapid Peroxidase Labeling Kit (manufactured by DOJINDO LABORATORIES, Product No. LK33). Then, luminescence was generated with the use of ImmunoStar (registered trademark) LD, and the resulting luminescence was detected on Image Quant LAS4000.

### (Measurement of Detected Intensity (Difference in Luminance))

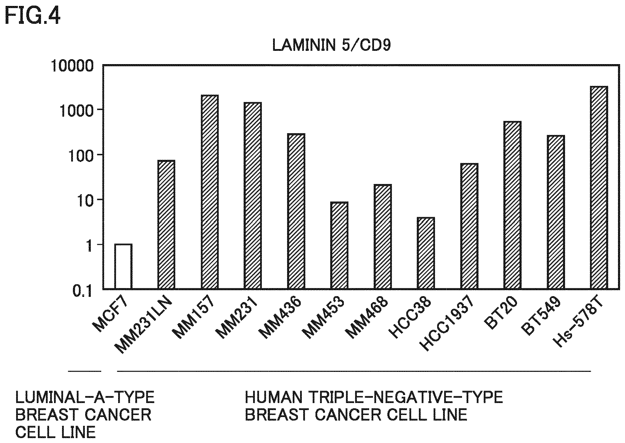

The total luminance of the immunochromatographic test strip at or around where the antibody was fixed as well as the total luminance of a non-luminescence area of the same size were measured by ImageJ, and from the difference between them (difference in luminance), the amount of CD9 expression and the amount of laminin 5 expression were determined. The amount of laminin 5 expression is divided by the amount of CD9 expression, and the resulting standardized value (laminin 5/CD9) is shown in Fig. 4. Laminin 5/CD9 was higher in all of the 11 types of triple-negative-type breast cancer cell lines thus investigated, than in the cell line that was not derived from triple-negative-type breast cancer (MCF7).

### <Experiment 3>

A biological specimen derived from a triple-negative-type breast cancer patient was investigated to see if laminin 5 expression was increased. In plasma specimens from three breast cancer patients (HER2-type, triple-negative-type, luminal-B-type) and three healthy individuals, detection of laminin 5 and CD9 was carried out by an immunochromatography method.

### (Detection of CD9 Expression Amount)

An immunochromatographic test strip was used, which had, in the same manner as in Experiment 2 above, a mouse anti-CD9 antibody fixed thereto and its membrane blocked with water-soluble polymer and BSA. Through this immunochromatographic test strip, 9 µL of a solution was developed, which contained plasma diluted three times with PBS containing 1% BSA and 0.05% Tween 20 and filtered through a syringe filter with a pore size of 0.22 µm. It was followed by developing a developer liquid containing a mouse anti-CD9 antibody to which HRP had been conjugated in the same manner as in Experiment 2. Then, luminescence was generated with the use of ImmunoStar (registered trademark) LD (Product No. 290-69904, manufactured by FUJIFILM Wako Pure Chemical Corporation), and the resulting luminescence was detected on Image Quant LAS4000.

### (Detection of Laminin 5 Expression Amount)

An immunochromatographic test strip was used, which had, in the same manner as in Experiment 2 above, a rabbit anti-LAMS antibody fixed thereto and its membrane blocked with water-soluble polymer and BSA. Through this immunochromatographic test strip, 90 µL of a solution was developed, which contained plasma diluted three times with PBS containing 1% BSA and 0.05% Tween 20 and filtered through a syringe filter with a pore size of 0.22 µm. It was followed by developing a developer liquid containing a rabbit anti-LAM5 antibody to which HRP had been conjugated in the same manner as in Experiment 2. Then, luminescence was generated with the use of ImmunoStar (registered trademark) LD (Product No. 290-69904, manufactured by FUJIFILM Wako Pure Chemical Corporation), and the resulting luminescence was detected on Image Quant LAS4000.

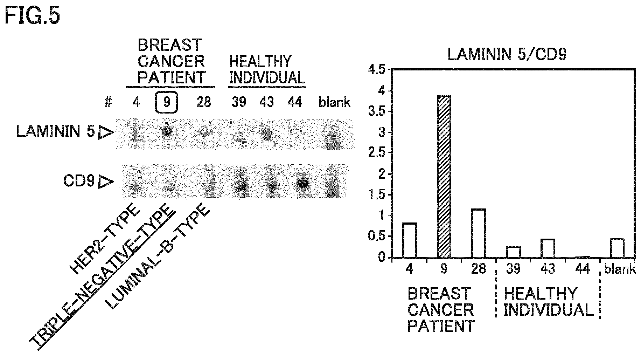

As a result, as seen in the left part of Fig. 5, laminin 5 and CD9 were detected. The total luminance of the immunochromatographic test strip at or around where the antibody was fixed as well as the total luminance of a non-luminescence area of the same size were measured by ImageJ, and from the difference between them (difference in luminance), the amount of laminin 5 expression and the amount of CD9 expression were determined. The luminance of laminin 5 is divided by the luminance of CD9, and the resulting value is shown in the right part of Fig. 5. Laminin 5/CD9 in the plasma from the breast cancer patients was higher than laminin 5/CD9 of the healthy individuals, and laminin 5/CD9 of the triple-negative-type breast cancer patient was higher than those of the other-subtype breast cancer patients.

### <Experiment 4>

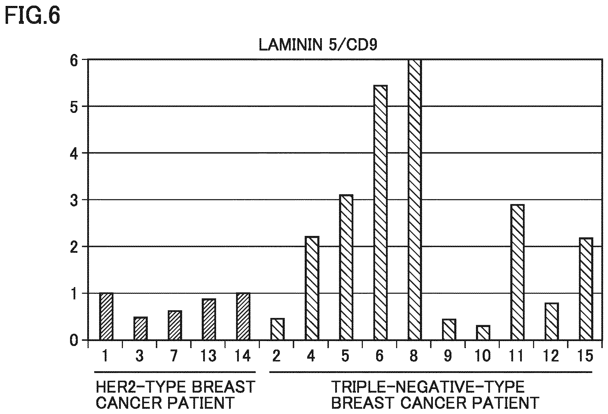

Further, for five HER2-type breast cancer patients and ten triple-negative-type breast cancer patients, the amount of CD9 expression and the amount of laminin 5 expression were measured. The experiment method was the same as in Experiment 3. The luminance of laminin 5 is divided by the luminance of CD9, and the resulting value is shown in Fig. 6. Laminin 5/CD9 in the plasma from six out often triple-negative-type breast cancer patients was higher, at least twice, than laminin 5/CD9 in the plasma from the HER2-type breast cancer patients. Moreover, based on the amount of laminin 5, the subtype (triple-negative-type) of breast cancer patients was successfully diagnosed with 100% specificity and 60% sensitivity. This suggested that laminin 5 is useful as a diagnostic marker for breast cancer subtyping.

### <Experiment 5>

More numbers of breast cancer patients and healthy individuals were investigated to see if laminin 5 expression in the breast cancer patients was increased. In plasma from 20 healthy individuals and 45 breast cancer patients, the amounts of expression of CD9 and laminin 5 were measured. The measurement method was the same as in Experiment 3. The subtypes and stages of the breast cancer patients are shown in Table 2 and Table 3.

**[Table 2]**

| Subtype | Number of patients | Proportion (%) |
|---|---|---|
| Luminal A | 20 | 44.5 |
| Luminal B | 8 | 17.8 |
| Her2 | 6 | 13.3 |
| Triple-negative | 11 | 24.4 |

**[Table 3]**

| Stage | Number of patients | Proportion (%) |
|---|---|---|
| I | 16 | 35.5 |
| II | 16 | 35.5 |
| III | 5 | 11.2 |
| IV | 8 | 17.8 |

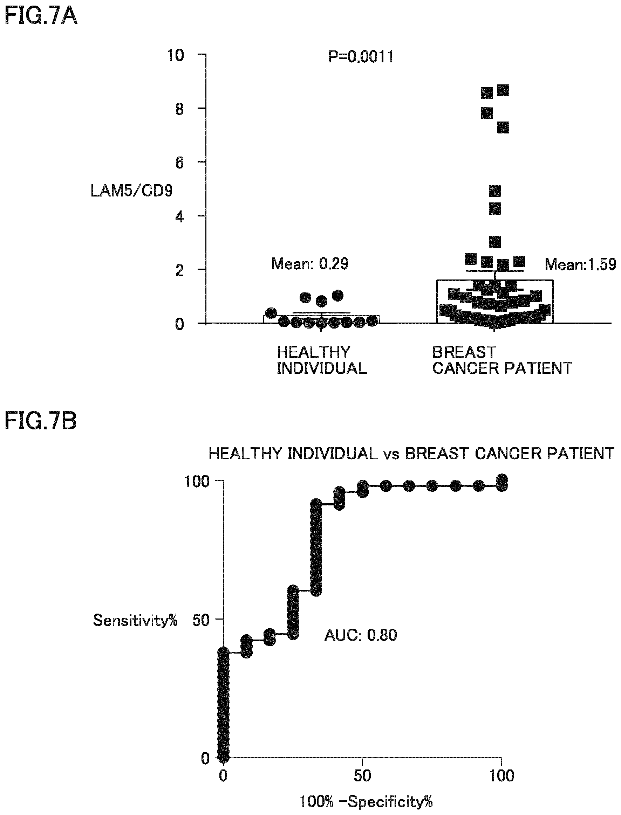

The luminance of laminin 5 is divided by the luminance of CD9, and the resulting standardized value (laminin 5/CD9) is shown in Fig. 7A. Laminin 5/CD9 in the breast cancer patients was higher than laminin 5/CD9 in the healthy individuals, with a statistic significance (p=0.0011). Fig. 7B shows a receiver operating characteristic (ROC) curve indicating the capability of laminin 5/CD9 of identifying breast cancer patients. The area under curve (AUC) was 0.80. From these, it is suggested that laminin 5 expression is high in the breast cancer patients and is highly capable of identifying breast cancer.

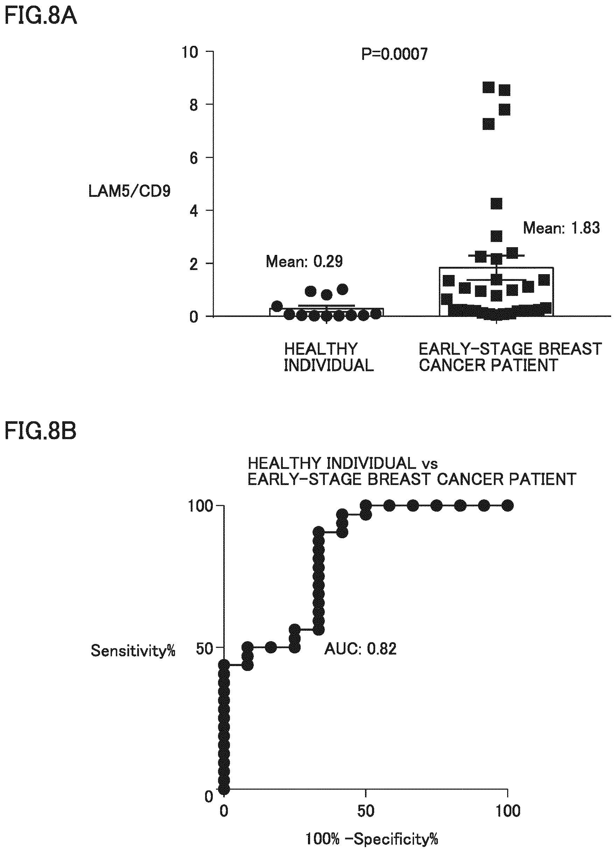

Comparison was performed between 32 early-stage breast cancer patients (Stages I and II) out of the breast cancer patients and 20 healthy individuals, in terms of the amount of laminin 5 expression. With reference to Fig. 8A, laminin 5/CD9 of the early-stage breast cancer patients was higher than laminin 5/CD9 of the healthy individuals, with a statistic significance (p=0.0007). Fig. 8B shows an ROC curve indicating the capability of laminin 5/CD9 of identifying early-stage breast cancer patients. AUC was 0.82. It is suggested that laminin 5 expression was high in the early-stage breast cancer patients and is highly capable of identifying early-stage breast cancer.

### <Experiment 6>

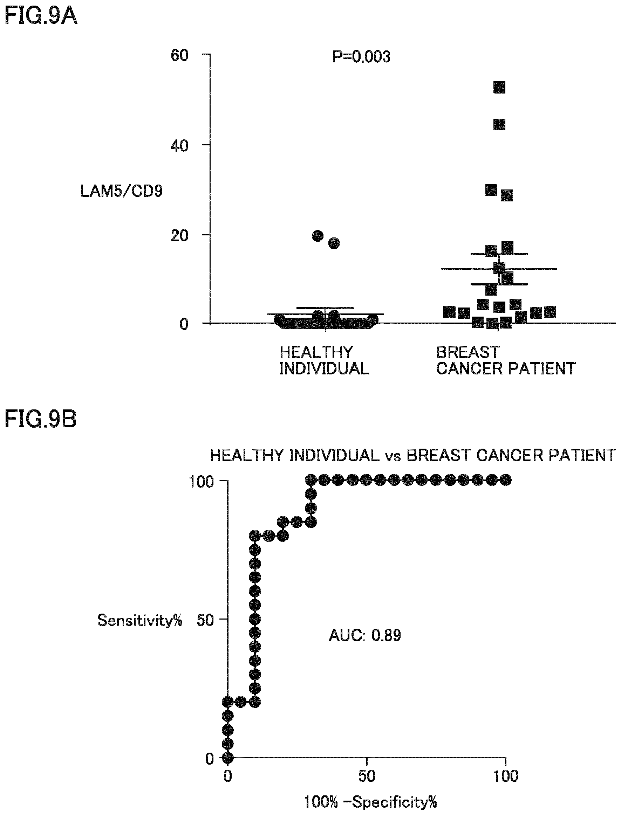

For a patient population different from Experiment 5 (20 breast cancer patients and 20 healthy individuals), usefulness of laminin 5 as a diagnostic marker for early-stage breast cancer was investigated. The stages of the breast cancer patients are shown in Table 4. In the plasma from the breast cancer patients and the healthy individuals, the amount of laminin 5 expression and the amount of CD9 expression were measured. The measurement method was the same as in Experiment 5. The calculated laminin 5/CD9 is shown in Fig. 9A. In the breast cancer patients, as compared to the healthy individuals, laminin 5 expression was high with a statistic significance (p=0.0003). AUC in the ROC curve for the breast cancer patients was 0.89 (Fig. 9B), indicating excellent capability of laminin 5 of identifying early-stage breast cancer. Thus, it was shown that laminin 5 is highly capable as a marker for identifying early-stage breast cancer.

**[Table 4]**

| Stage | Number of patients | Proportion (%) |
|---|---|---|
| I | 10 | 50 |
| II | 6 | 30 |
| III | 3 | 15 |
| IV | 1 | 5 |

### [Aspects]

As will be appreciated by those skilled in the art, the above-described example embodiments and Examples are specific examples of the below aspects.

### (Item 1)

A method of assisting breast cancer diagnosis according to an aspect comprises: a measuring step for measuring an amount of laminin 5 or laminin β3 contained in a specimen; and an information-providing step for providing information for breast cancer diagnosis based on the amount of laminin 5 or laminin β3 thus measured.

With the diagnosis assistance method according to Item 1, it is possible to provide information for breast cancer diagnosis in an easy and simple manner.

### (Item 2)

In the diagnosis assistance method according to Item 1, the information for breast cancer diagnosis includes information for diagnosis of presence or absence of breast cancer.

With the diagnosis assistance method according to Item 2, it is possible to provide information for diagnosis of presence or absence of breast cancer in an easy and simple manner.

### (Item 3)

In the diagnosis assistance method according to Item 1 or Item 2, the information for breast cancer diagnosis includes information for diagnosis of breast cancer subtype.

With the diagnosis assistance method according to Item 3, it is possible to provide information for diagnosis of breast cancer subtype in an easy and simple manner.

### (Item 4)

In the diagnosis assistance method according to any one of Item 1 to Item 3, the information for breast cancer diagnosis includes information for diagnosis of breast cancer stage.

With the diagnosis assistance method according to Item 4, it is possible to provide information for diagnosis of breast cancer stage in an easy and simple manner.

### (Item 5)

In the diagnosis assistance method according to any one of Item 1 to Item 4, in the measuring step, the amount of laminin 5 or laminin β3 is measured by an immunological measurement method or a mass spectrometry method.

With the diagnosis assistance method according to Item 5, it is possible to accurately measure the amount of laminin 5 and laminin β3.

### (Item 6)

In the diagnosis assistance method according to Item 5, the immunological measurement method is an immunochromatography method.

With the diagnosis assistance method according to Item 6, it is possible to measure the amounts of laminin 5 and laminin β3 in a fast, easy, and simple manner.

### (Item 7)

In the diagnosis assistance method according to Item 5 or 6, an antibody used in the immunological measurement method is labelled with metal particles or with an enzyme.

When the antibody is labelled with an enzyme, the amount of laminin 5 or laminin β3 can be measured with high sensitivity. When the antibody is labelled with metal particles, the amount of laminin 5 or laminin β3 can be detected by means of visible light (absorption).

### (Item 8)

In the diagnosis assistance method according to Item 7, the metal particles are detected by inductively coupled plasma mass spectrometry (ICP-MS).

With the diagnosis assistance method according to Item 8, it is possible to detect the metal particles even when they are in a small amount, allowing for highly-sensitive measurement of the amount of laminin 5 or laminin β3.

### (Item 9)

In the diagnosis assistance method according to any one of Item 1 to Item 8, in the information-providing step, the amount of laminin 5 or laminin β3 is standardized based on an amount of exosomes.

With the diagnosis assistance method according to Item 9, it is possible to enhance the accuracy of the information for breast cancer diagnosis.

### (Item 10)

In the diagnosis assistance method according to Item 9, the amount of exosomes is measured with use of CD9 and used for standardizing the amount of laminin 5 or laminin β3.

With the diagnosis assistance method according to Item 10, it is possible to enhance the accuracy of the information for breast cancer diagnosis.

### (Item 11)

In the diagnosis assistance method according to any one of Item 1 to Item 10, the specimen is a biological specimen collected from a subject.

With the diagnosis assistance method according to Item 11, it is possible to provide information for breast cancer diagnosis of the subject.

### (Item 12)

In the diagnosis assistance method according to any one of Item 1 to Item 11, the specimen is blood collected from a human individual.

With the diagnosis assistance method according to Item 12, it is possible to provide information for breast cancer diagnosis of the human.

### (Item 13)

In the diagnosis assistance method according to any one of Item 1 to Item 12, the specimen is a purified exosome fraction.

With the diagnosis assistance method according to Item 13, it is possible to lower the amount of foreign matter in the specimen, leading to enhanced accuracy of the measurement.

### (Item 14)

A test kit for breast cancer according to an aspect comprises an anti-laminin 5 antibody or an anti-laminin β3 antibody.

With the test kit for breast cancer according to Item 14, it is possible to provide information for breast cancer diagnosis in an easy and simple manner by measuring the amount of laminin 5 or laminin β3 contained in a specimen.

### (Item 15)

In the test kit according to Item 14, the anti-laminin 5 antibody or the anti-laminin β3 antibody is labelled with metal particles or with an enzyme.

When the antibody is labelled with an enzyme, the amount of laminin 5 or laminin β3 can be measured with high sensitivity. When the antibody is labelled with metal particles, laminin 5 or laminin β3 can be detected by means of visible light (absorption).

### (Item 16)

The test kit according to Item 14 or Item 15 comprises an immunochromatographic test strip.

With the test kit according to Item 16, it is possible to measure the amount of laminin 5 or laminin β3 in a fast, easy, and simple manner.

### (Item 17)

A breast cancer diagnostic marker according to an aspect consists of laminin 5 or laminin β3 contained in a specimen.

With the diagnostic marker according to Item 17, it is possible to provide information that is useful for breast cancer diagnosis, in an easy and simple manner.

### (Item 18)

A measurement method according to an aspect measures the amount of laminin 5 or laminin β3 contained in a specimen.

With the measurement method according to Item 18, it is possible to determine the amount of laminin 5 or laminin β3 contained in the specimen. The amount of laminin 5 or laminin β3 can be used as information for judging if the specimen was derived from breast cancer.

### (Item 19)

A breast cancer diagnostic method according to an aspect comprises judging that a specimen was derived from breast cancer when the amount of laminin 5 or laminin β3 contained in the specimen is equal to or higher than a predetermined value.

With the breast cancer diagnostic method according to Item 19, it is possible to diagnose if the specimen was derived from breast cancer, in an easy and simple manner.

### (Item 20)

Use of an anti-laminin 5 antibody or an anti-laminin β3 antibody according to an aspect is for breast cancer diagnosis.

With the use according to Item 20, it is possible to provide diagnosis of, or information for diagnosis of, breast cancer.

### (Item 21)

Use of an anti-laminin 5 antibody or an anti-laminin β3 antibody according to an aspect is for producing a test kit for breast cancer.

With the use according to Item 21, it is possible to produce a test kit for breast cancer.

It should be construed that all the embodiments and Examples disclosed herein are given by way of illustration in all respects, not by way of limitation. It should also be construed that the scope of the present invention is interpreted by the terms of the appended claims, not by the above description, and encompasses all modifications and variations equivalent in meaning and scope to the claims.

### REFERENCE SIGNS LIST

10 immunochromatographic test strip, 11 membrane, 12 water-absorbing pad, 13 capture antibody, 14 sample pad, 15 conjugate pad, 16 detection target, 17 detection antibody.

## Claims

1. A method of assisting breast cancer diagnosis, comprising:
measuring an amount of laminin 5 or laminin β3 contained in a specimen; and
based on the amount of laminin 5 or laminin β3 thus measured, providing information for breast cancer diagnosis.

2. The method according to claim 1, wherein the information for breast cancer diagnosis includes information for diagnosis of presence or absence of breast cancer.

3. The method according to claim 1 or 2, wherein the information for breast cancer diagnosis includes information for diagnosis of breast cancer subtype.

4. The method according to any one of claims 1 to 3, wherein the information for breast cancer diagnosis includes information for diagnosis of breast cancer stage.

5. The method according to any one of claims 1 to 4, wherein in the measuring, the amount of laminin 5 or laminin β3 is measured by an immunological measurement method or a mass spectrometry method.

6. The method according to claim 5, wherein the immunological measurement method is an immunochromatography method.

7. The method according to claim 5 or 6, wherein an antibody used in the immunological measurement method is labelled with metal particles or with an enzyme.

8. The method according to claim 7, wherein the metal particles are detected by inductively coupled plasma mass spectrometry (ICP-MS).

9. The method according to any one of claims 1 to 8, wherein in the providing information, the amount of laminin 5 or laminin β3 is standardized based on an amount of exosomes.

10. The method according to claim 9, wherein the amount of exosomes is measured with use of CD9 and used for standardizing the amount of laminin 5 or laminin β3.

11. The method according to any one of claims 1 to 10, wherein the specimen is a biological specimen collected from a subject.

12. The method according to any one of claims 1 to 11, wherein the specimen is blood collected from a human individual.

13. The method according to any one of claims 1 to 12, wherein the specimen is a purified exosome fraction.

14. A test kit for breast cancer comprising an anti-laminin 5 antibody or an anti-laminin β3 antibody.

15. The test kit according to claim 14, wherein the anti-laminin 5 antibody or the anti-laminin β3 antibody is labelled with metal particles or with an enzyme.

16. The test kit according to claim 14 or 15, comprising an immunochromatographic test strip.
